# EUROPEAN PATENT APPLICATION

(11) **EP 1 358 860 A2**
(43) Date of publication of application: **05.11.2003**
(21) Application number: 03252699.8
(22) Date of filing: 29.04.2003
(51) Int. Cl.: A61F 2/30, A61F 2/36, A61F 2/38

(54) **A modular orthopaedic implant system**

(30) Priority: 30.04.2002 US 135791
(71) Applicant: DePuy Orthopaedics, Inc., Warsaw, IN 46582 (US)
(72) Inventor: Hazebrouck, Stephen A., Winona Lake, IN 46590 (US); Ruhling, Marc E., Goshen, IN 46528 (US); Deeter, Nick A., Warsaw, IN 46582 (US); Lester, Mark B., Warsaw, IN 46580 (US); Ferguson, Joe W., Issaquah, WA 98029 (US)
(74) Representative: Belcher, Simon James

(57) **Abstract**

A modular implant system for limb preservation comprises modular components which can be combined to perform proximal, distal, mid-shaft or total long bone replacement. Each replacement component is connected by a Morse taper engagement. The Morse taper engagement can be augmented by interlocking anti-rotation features between adjacent components. Certain components are configured to form part of a skeletal joint, while other components are configured for engagement within existing portions of the long bone. A number of segmental components operate as spacers for spanning the length of a removed section of bone. Certain segmental components are provided in several lengths, with one group of lengths increasing in 5 mm increments. Combinations of the segmental components can accommodate replacement lengths from 25 mm to 170 mm in 5 mm increments.

## Description

The present invention relates to prosthetic systems for the replacement of limbs or portions thereof. More particularly, the invention concerns a modular orthopaedic implant system that can be used in the extremities that have experienced bone loss or significant, irreparable bone trauma.

Artificial or prosthetic joints of the extremities are well-known. Many of prosthetic joints are modular, meaning that they include a selection of different components to account for differences in patient anatomy or surgical procedures. For example, US-5314479 discloses a modular shoulder prosthesis that includes an array of selectable stems, bodies, collars and head members. The disclosed modular prosthesis allows the orthopaedic surgeon to assemble a custom prosthetic joint by selecting different sizes, shapes and orientations of the individual joint components.

Certain orthopaedic prosthetic devices include implants or stems configured to reside within the intra medullary canal of a long bone. For instance, the modular prosthesis disclosed in the'479 document includes a stem sized to be received within the humerus and held in place with bone cement. Two differently sized stems are provided, each having different lengths and diameters to accommodate differently sized bones. In place of a kit with differently sized stems, other systems utilize variable length stems. One such adjustable length implant includes threaded telescoping portions, such as the implant shown in US-5358524. This particular device is intended for adjustment over time as the limb grows. An adjustable artificial femoral diaphysis is disclosed in US-4384373 that employs a similar threaded telescoping arrangement to accommodate any length femur.

These modular or adjustable devices all require a certain amount of viable bone, and are not suited for limb preservation when a significant portion of a long bone has been lost due to tumour, end-stage revision or severe trauma. In cases involving significant bone loss, very little is presently available to preserve the damaged limb and the opportunity for mobility of the patient.

Consequently, there is a need for a system that allows preservation of a badly damaged limb, and more importantly for protecting patient mobility in the wake of severe damage to a limb. There is a further need for a system that can accommodate differences in patient anatomy, as well as differences in the traumatized limb to be repaired.

In order to address these needs, the present invention provides a comprehensive modular implant system designed to meet the challenges of limb-sparing surgery. In one embodiment, the comprehensive modular orthopaedic implant system comprises at least three components, with at least one component configured for replacement of a portion of the bone of a limb of a patient, and at least another component configured for implantation within a remaining portion of the bone. The implant system further includes a male-female engagement between adjacent ones of the components. In a preferred embodiment, the male-female engagement is a press-fit engagement, and most preferably a Morse taper engagement.

The present invention contemplates a limb preservation system that includes a comprehensive set of modular implants capable of addressing a wide range of orthopaedic conditions. Components of the system can be combined in a variety of ways to account for variations in patient anatomy and differences in bone or limb trauma. For instance, the comprehensive modular implant system of the present invention can be employed as a replacement for the proximal or distal femur, total femur, proximal tibia or a long bone mid-shaft. The particular modular components can be selected after consideration of the limb trauma, and more specifically in view of the degree and type of bone loss involved, such as might occur due to tumour, end-stage revision or trauma.

In one aspect of the invention, at least one component includes a joint component configured to form part of a skeletal joint. For instance, the modular implant system contemplates a proximal femoral replacement for use with a hip joint prosthesis, as well as a distal femoral replacement and a proximal tibial replacement for a knee joint prosthesis.

In a further aspect of the invention, the comprehensive modular orthopaedic implant system includes a stem configured for implantation within the intra medullary canal of the remaining bone. This stem can be used to replace a severely damaged portion of the long bone adjacent a particular joint being replaced. This modular stem component provides the orthopaedic surgeon with the ability to assess how much bone needs to be replaced and then assemble a modular construct meeting that need.

As an attribute of the overall modularity of the inventive system, a series of modular components can be combined to replace an entire long bone, such as a femur. The modular system thus include an array of segmental components that can act as spacers to fill the length of removed bone. These segmental component can be selected from a plurality of segmental components having different lengths. A given construct may include multiple segmental component selected from the plurality of segmental components having different lengths.

In one inventive feature, the plurality of segmental components can include a first group of segmental components having lengths of 25 mm, 30 mm, 35 mm, 40 mm and 45 mm. In another feature, a second group of segmental components can be provided having lengths of 65 mm, 85 mm, 105 mm and 125 mm. In a particular modular construct, a segmental component selected from the first group can be combined with a segmental component selected from either the first group or the second group to produce a combined length from 50 mm to 170 mm in 5 mm increments.

The invention contemplates various combinations of modular components for addressing different orthopaedic surgical needs. For instance, in one embodiment, the construct includes only a pair of stems configured for implantation within proximal and distal portions of the bone, and a spacer disposed between the pair of stems. Another construct utilizes a first joint component configured to form part of a skeletal joint at the proximal end of the limb, a second joint component configured to form part of a skeletal joint at the distal end of the limb, and at least two intermediate segmental components spanning the length of the limb between said first and second joint components, all incorporating a male-female engagement between adjacent one of the components.

Embodiments of the invention will now be described by way of example with reference to the accompanying drawings, in which:
FIG. 1 is a perspective view of a proximal femoral replacement assembled from components of the comprehensive modular limb preservation system in accordance with one embodiment of the present invention.
FIG. 2 is a perspective view of a distal femoral replacement assembled from components of the comprehensive modular limb of the present invention.
FIG. 3 is a perspective view of a total femoral replacement assembled from components of the comprehensive modular limb of the present invention.
FIG. 4 is a perspective view of a proximal tibial replacement assembled from components of the comprehensive modular limb of the present invention.
FIG. 5 is a perspective view of a mid-shaft femoral replacement assembled from components of the comprehensive modular limb of the present invention.
FIG. 6 is an exploded perspective view of the components of the proximal femoral replacement shown in FIG. 1.
FIG. 7 is an exploded perspective view of the components of the distal femoral replacement shown in FIG. 2.
FIG. 8 is an exploded perspective view of the components of the total femoral replacement shown in FIG. 4.
FIG. 9 is an exploded perspective view of the components of the mid-shaft femoral replacement shown in FIG. 5.
FIG. 10 is an exploded perspective view of the components of the proximal tibial replacement shown in FIG. 3.
FIG. 11 is an enlarged perspective view of the proximal femoral component employed in the proximal femoral replacement shown in FIGS. 1 and 6 and in the total femoral replacement shown in FIGS. 3 and 10.
FIG. 12 is a side view of the proximal femoral component shown in FIG. 11.
FIG. 13 is an enlarged perspective view of a male-male segmental component employed in the total femoral replacement shown in FIGS. 3 and 10.
FIG. 14 is a side view of the male-male segmental component shown in FIG. 13.
FIG. 15 is an enlarged perspective view of a male-female segmental component employed in the proximal femoral replacement shown in FIGS. 1 and 6 and in the total femoral replacement shown in FIGS. 3 and 10.
FIG. 16 is an enlarged cross-sectional view of an end of the male-female segmental component shown in FIG. 15.
FIG. 17 is an enlarged perspective view of a distal femoral component employed in the distal femoral replacement shown in FIGS. 2 and 7 and in the total femoral replacement shown in FIGS. 3 and 10.
FIG. 18 is a side view of the distal femoral component shown in FIG. 17.
FIG. 19 is an enlarged perspective view of a tibial insert bearing and tibial tray components employed in the distal femoral replacement shown in FIGS. 2 and 7, in the total femoral replacement shown in FIGS. 3 and 10, and in the proximal tibial replacement shown in FIGS. 4 and 8.
FIG. 20 is a side cross-sectional view of the components shown in FIG. 19.
FIG. 21 is an enlarged perspective view of one embodiment of a stem for engagement within the intra medullary canal of a long bone for use with the replacements shown in the figures.
FIG. 22 is an enlarged perspective view of an alternative embodiment of a stem for engagement within the intra medullary canal of a long bone for use with the replacements shown in the figures.
FIG. 23 is a side view of a proximal tibial component employed in the proximal tibial replacement shown in FIGS. 4 and 8.
FIG. 24 is a side cross-sectional view of the proximal tibial component shown in FIG. 23.
FIG. 25 is an enlarged perspective view of a female-female segmental component employed in the mid-shaft femoral replacement shown in FIGS. 5 and 9.
FIG. 26 is an enlarged cross-sectional view of an end of the female-female segmental component shown in FIG. 25.

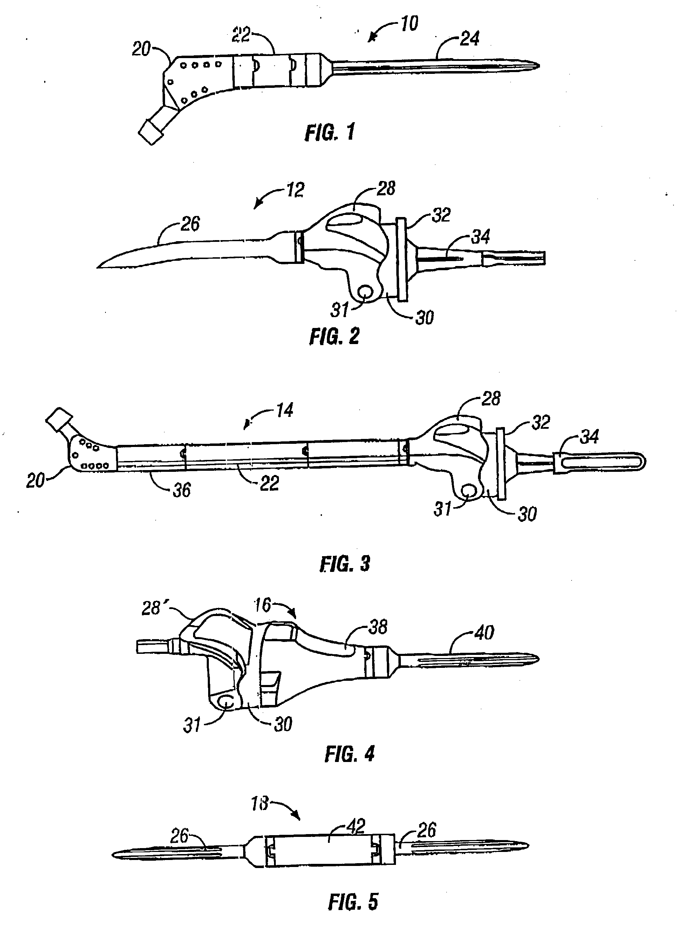

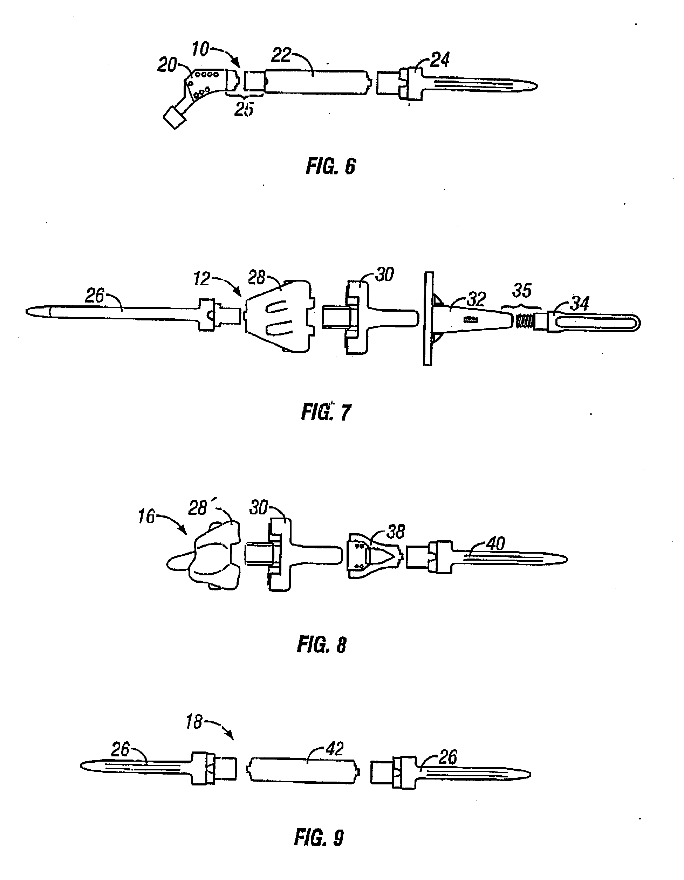

Referring to FIGS. 1-5, various combinations of the modular components are illustrated for use in different indications. Thus, where the proximal end of a long bone is removed, a proximal replacement, such as the proximal femoral replacement 10 shown in FIGS. 1 and 6, can be utilized. This proximal replacement 10 includes a proximal femoral component 20, a male-female segmental component 22 and a femoral stem extension 24. The proximal femoral component 20 is configured to replace the proximal end of the bone, while the segmental component 22 is sized to replace a segment of bone adjacent the proximal end.

On the other hand, a distal replacement can be fashioned from the components of the present comprehensive modular implant system. For instance, a distal femoral replacement 12, illustrated in FIGS. 2 and 7, includes a femoral stem 26, a distal femoral component 28, a tibial insert bearing 30, a tibial tray 32, and a stem extension 34.

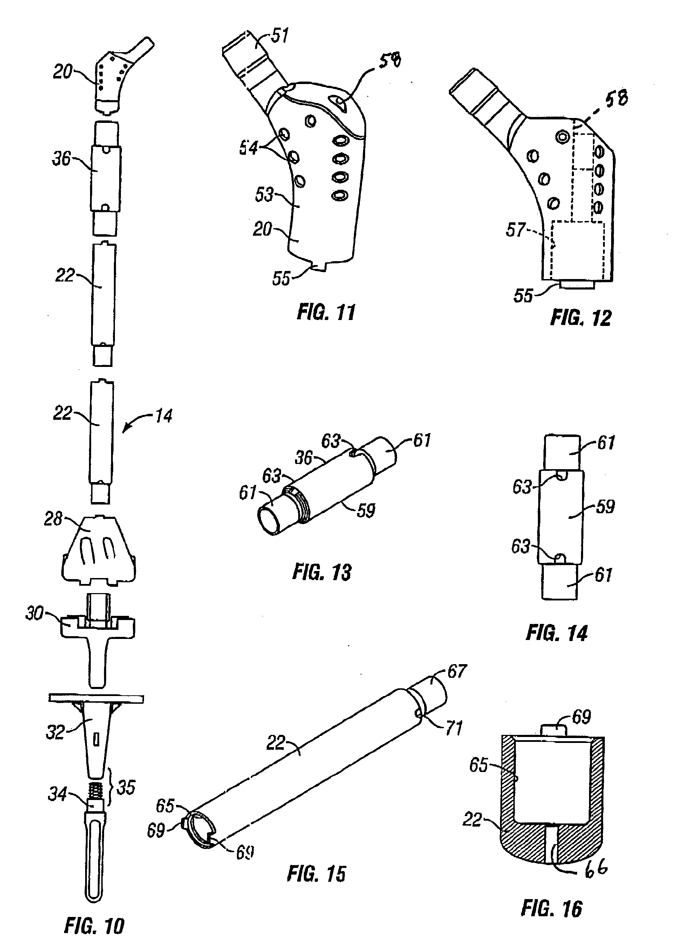

The modular system can be configured for total long bone replacement. In one embodiment, as shown in FIGS. 3 and 10, a total femoral replacement 14 includes some of the components of the two replacements just described. For instance, the total replacement can include a proximal femoral component 20, distal femoral component 28, tibial insert bearing 30, tibial tray 32 and stem extension 34. In order to accommodate the length of the particular long bone being replaced (e.g., the femur in the illustrated case), the total replacement 14 can include a number of male-female segmental components 22. In addition, in order to interface the male-female components 22 with the proximal femoral component 20, a male-male segmental component 36 can be provided.

Referring to FIGS. 4 and 8, a proximal tibial replacement 16 can include a modified distal femoral component 28' and a tibial insert bearing 30, usable with the replacements discussed above. In addition, the replacement 16 can include a proximal tibial component 38 and a stem 40.

In some cases, the proximal and distal ends of the limb are intact, but the mid-shaft portion of the long bone is damaged beyond repair. In these instances, the modular implant system of the present invention provides a mid-shaft replacement 18 that can include opposite stems 26 joined by a female-female segmental component 42. The segmental component 42 can essentially operate as a spacer between the two stems 26 to span a space left by a removed portion of bone.

As best seen in the exploded diagrams of FIGS. 6-10, the comprehensive modular implant system utilizes a series of male-female connections 25 between adjacent components, as shown in FIG. 6. In the preferred embodiment, these connections are press-fit connections. Most preferably, the press-fit connections are Morse taper connections, well-known in the design of orthopaedic implants as providing a strong and stable union between adjacent implant components. Each Morse taper connection includes a male and a female element.

The arrangement of the male and female elements for each connection shown in FIGS. 6-10 can follow the convention depicted in the figures, or can implement a different convention as to which of the adjacent components includes the male element and which includes the female element. In a preferred convention, the proximal or distal components, such as the proximal femoral component 20, distal femoral component 28 or proximal tibial component 38, only include female Morse taper elements. Likewise, the stems, such as stems 26 and 40, or stem extensions, such as extension 24, only include a male Morse taper element. It is believed that this convention makes assembly of the various components easier, and is preferable from a strength standpoint.

Referring now to FIGS. 11-26, details will be described for the various components of the comprehensive modular implant system of the present invention. The proximal femoral component 20, shown in FIGS. 11 and 12, can be of a known size and body style for incorporation into a hip replacement procedure. The component 20 can include a neck 51 configured to mate with known femoral heads, such as the device supplied by DePuy Orthopaedics Inc under the trade mark ARTICUL/EZE. The body 53 of the component can include holes 54 for soft tissue re-attachment.

In accordance with the present invention, the conventional proximal femoral component is modified to include a male-female connection element, such as a Morse taper bore 57 at the distal end of the component 20. In addition, the component includes a number of anti-rotation tabs 55 projecting from the distal end. The bore 57 and tabs 55 mate with complementary elements, as described herein.

With reference to the total femoral replacement 14 shown in FIG. 10, the next component is the male-male segmental component 36, which is shown in detail in FIGS. 13 and 14. In particular, the segmental component 36 has a diameter that is sized to replace a bone segment, such as a segment of the femur. The component 36 includes an elongated body 59 that terminates at its ends in a male connection element, such as a Morse taper post 61. Each post 61 is sized to be received within a bore, such as bore 57 in the proximal femoral component 20. Thus, the length of the Morse taper post 61 is calibrated to the depth of the Morse taper bore 57. In fact, this convention can be, and is most preferably, applied at all Morse taper connections in the modular implant system of the present invention. Following this sizing convention allows any component having a male Morse taper post to mate with any other component having a female Morse taper bore. In a specific embodiment, each Morse taper bore has a depth of about 24.1 mm(0.950 inch), while the post can have a length of about 20.3 mm (0.800 inch).

In order to accommodate the mating of the Morse taper components, the bore 57 of the proximal femoral component 20 communicates with a bore 58, as shown in FIGS. 11 and 12. In normal use, the bore 58 can receive a tool for guiding the component into its implanted position. However, when a post 61 (FIG.13) is introduced into the bore 57, the bore 58 acts as a vent to prevent air from being trapped within the bore behind the post 61.

Referring again to FIGS. 13 and 14, the body 59 of the male-male segmental component 36 defines a number of anti-rotation notches 63 adjacent the corresponding post 61. Each notch 63 is sized to receive a tab 55 therein when the two components are connected together. Thus, the interdigitation of the tabs 55 with the notches 63 prevent relative rotation between the connected components. Preventing this relative rotation helps maintain the union between the two components at the Morse taper connection. In a specific embodiment, the interlocking tabs and notches can have lengths of about 0.3 mm (0.120 inch). Also in the specific embodiment, two such interlocking elements can be situated at diametrically opposite positions, although greater numbers of tabs and notches can be utilized.

As shown in FIG. 10, the total femoral replacement 14 includes a second type of segmental component, namely the segmental components 22. As seen in FIG. 10, these components 22 are longer than the male-male component 36. Moreover, these segmental components 22 have a male connection element at one end and a female connection element at the opposite end. Thus, as shown in more detail in FIGS. 15 and 16, the segmental component 22 includes a Morse taper bore 65 at one end and a Morse taper post 67 at its opposite end. In addition, the component includes a number of anti-rotation tabs 69 at the bore end and a number of anti-rotation notches 71 at the post end. The Morse elements and interlocking elements can be configured and dimensioned as described above.

Since the segmental component 22 incorporates a Morse taper fit, the taper bore 65 is provided with a vent bore 66, as shown in FIG.16. The vent bore can extend along the entire length of the component 22, opening at the taper post 67. As with the vent bore 58 described above, the vent bore prevents air from being trapped behind the mating Morse taper component.

In accordance with the present invention, the male-female segmental components 22 contribute to one aspect of the modularity of the present system. As illustrated in FIG. 10, two such components 22 are combined to provide a total femoral replacement 14 that is long enough to replace the entire femur when combined with the other replacement components. In prior systems, length adjustments for long bone replacements or intra medullary stems have required a telescoping and/or threaded connection. With the present invention, an array of differently sized segmental components 22 can be provided that can be combined in any manner necessary to nearly exactly match any length femur.

In one aspect of the invention, the array of segmental components 22 is provided in 5 mm increments of increasing length. Thus, in a specific embodiment, the array includes male-female segmental components having lengths of 25, 30, 25, 40, and 45 mm. In the first instance, these lengths permit replacement of varying lengths of the long bone adjacent the proximal or distal ends. For instance, the segmental component 22 is used in the proximal femoral replacement 10, so the length of the component can be selected based on the amount of bone removed at the proximal end of the limb.

In a second instance, the lengths can be combined for a total long bone replacement, such as the total femoral replacement 14. In addition to the aforementioned lengths, the array can include longer components 22 having lengths of 65, 85, 105 and 125 mm. It can be appreciated that this selection of segmental component lengths permits combined lengths increasing in 5 mm increments from the 45mm component to the 125mm component. For instance, a 50mm length can be achieved with two 25mm components, while a 110mm length uses an 85mm and a 25mm segmental component. Virtually any length of long bone can be accommodated by combining only two of the segmental components from the array of sizes just described. Viewed from another angle, a kit containing the components of the comprehensive modular implant system of the present invention only needs to include one of each of the sizes mentioned above to replace the bone virtually any limb.

In view of the "mix and match" aspect of the array of segmental components 22, the need for the male-male segmental component 36. can be appreciated. Specifically, since any of the segmental components 22 can be combined and then mated with other replacement components, it is important that the components 22 include a male and a female connection element. If it is desirable to include only female Morse taper bores in the proximal or distal femoral/tibial components, then it is inevitable that a like Morse element will be juxtaposed when a replacement stack is created. Thus, the male-male segmental component 36 can be interposed to provide a connection for juxtaposed female elements.

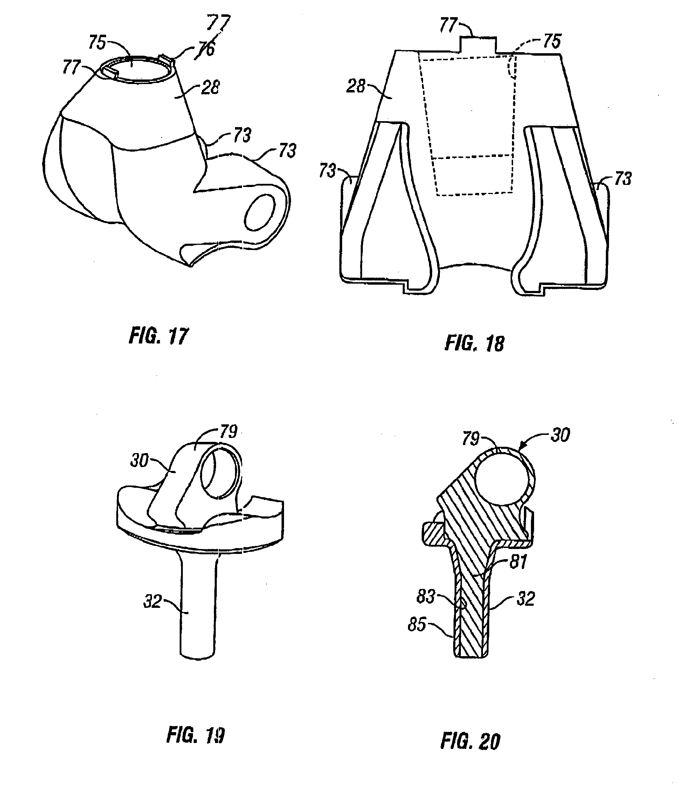

Returning to FIG. 10, the total replacement 14 includes a distal femoral component 28 that forms part of a prosthetic knee joint. As shown in FIGS. 17 and 18, the femoral component 28 is of conventional design, including a pair of hinge arms 73. In a modification for the present invention, the component 28 further includes a female connection element, such as a bore 75, and a number of anti-rotation tabs 77, all as described above.

The distal femoral component 28 mates with a tibial insert bearing 30, shown in detail in FIGS. 19 and 20. The insert bearing 30 includes a hinge element 79 that is configured in a known manner to engage the hinge arms 73. Each of the hinge pieces includes a bore to receive a hinge pin 31 (FIGS. 2-4) which can again be of conventional design. The tibial insert bearing 30 mates with a tibial tray 32 in a known manner. Specifically, the bearing 30 includes a mating stem 81 that engages a mating bore 83 in the tibial tray. The bore 83 extends along the length of the Morse taper post 85 that is used to mate with a subsequent component of the modular system.

As with the other components, the knee joint elements represented by the components 28, 30 and 32 can all be formed substantially as any known knee joint. For instance, the components can be part of the knee implant supplied by DePuy Orthopaedics Inc under the trade mark S-ROM. The revision tray 32 can be that sold under the trade mark MBT by DePuy Orthopaedics Inc. As shown in FIGS. 7 and 10, the revision tray 32 mates with a tibial stem extension 34 by way of a threaded connection 35. Other types of connection are contemplated with appropriate modification to the tibial revision tray 32.

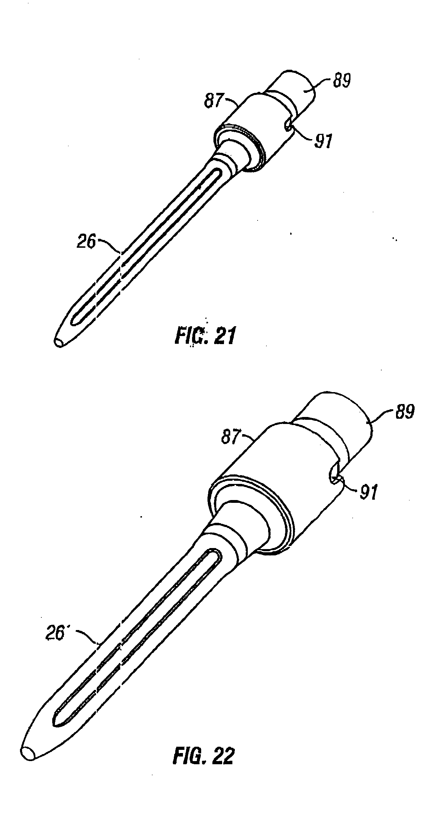

The modular implant system of the present invention is capable of certain of the replacement configurations that utilize elongated stems for fixation within the intra medullary canal of the associated long bone. Thus, a kit for this modular system can include femoral stems 26 and 26' as illustrated in FIGS. 21 and 22. The two stems have different lengths and diameters to accommodate differently sized bones. However, each stem 26, 26' includes a head portion 87 from which extends a male connection element Morse taper post 89. A number of interlocking notches 91 are also formed in the head portion so that the stem can be connected to another of the modular components. The stem 40 shown in FIG. 8 can be similarly configured.

It can be appreciated that any of the stems or stem extensions can have an exterior surface prepared for cementing within an existing bone. Thus, the stems or extension can include channels or openings for receiving bone cement and providing a firm fixation to the surrounding bone. These same components can be porous coated using known technology. On the other hand, components that are not intended for fixation to surrounding tissue, such as the segmental components, are preferably treated to have a very smooth, non-adherent surface, such as through bead-blasting and polishing.

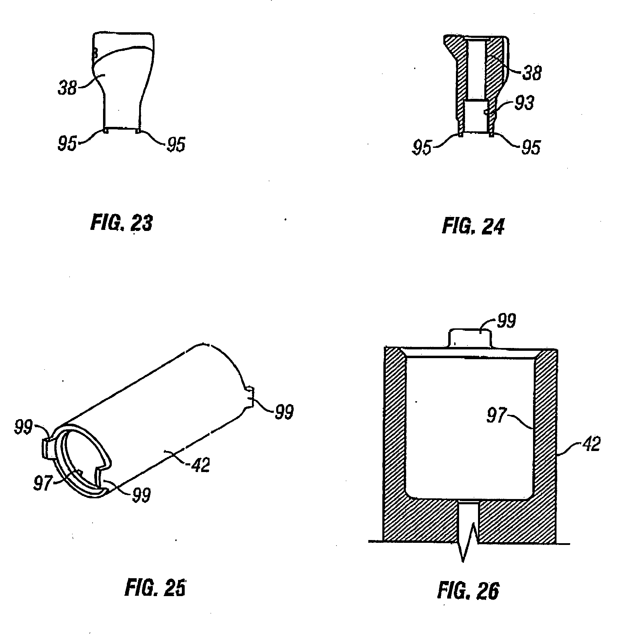

The comprehensive nature of the modular implant system of the present invention contemplates a kit that can be used for proximal tibial replacement as part of a prosthetic knee joint. Thus, as shown in FIGS. 4 and 8, a proximal tibial replacement includes a proximal tibial component 38. As shown in FIGS. 23 and 24, this component 38 can have the form of a conventional tibial replacement. This convention design is modified in accordance with the present invention to incorporate a female Morse taper bore 93 and associated interlocking anti-rotation tabs 95. This portion of the component 38 is configured to mate with a tibial stem 40 that includes the features of the stem 26 shown in FIG. 21.

A further feature of the modularity of the present invention is the ability to replace only a segment of a long bone, while leaving the proximal and distal ends intact. Such a replacement might occur where a limb has suffered a severe trauma at the mid-section of the bone, requiring replacement of that section. In order to address this trauma indication, the present invention provides a mid-shaft replacement 18, shown in FIGS. 5 and 9. This replacement includes two stems 26 configured to be implanted into the medullary canal of the intact proximal and distal bone sections.

The heart of this mid-shaft replacement 18 is the female-female segmental component 42. In accordance with convention established above, this component 42 includes a female connection element, such as Morse taper bore 97, and anti-rotation tabs 99 at each end of the component. Due to the nature of mid-shaft bone replacement, it is preferable to offer the segmental component 42 in a single length. Most preferably, the component 42 has a length of 55mm, which is believed to be optimum for most mid-shaft replacement procedures. Any greater damage to the bone would usually require replacement of the entire bone, using the replacement assembly shown in FIGS. 3 and 10.

The components of the modular implant system of the present invention can be formed of conventional bio-compatible metals or suitably strong materials. For instance, the proximal femoral and proximal tibial components 20 and 38, respectively, can be formed of a titanium alloy. The distal femoral component 28 and the stems and extensions can be formed of a cobalt-chromium alloy. Most preferably, the segmental components 22, 36 and 42 are formed of a titanium alloy.

The stem and extensions can be cemented or porous coated and can be straight or bowed depending upon the preferred application. These components can be offered in conventional lengths and diameters, with femoral straight stems in 100 mm and 125 mm length and curved stems in 150 mm and 200 mm lengths. The diameters can range from 9 mm to 18.5 mm. Tibial stems can have a conventional diameter of 13 mm and lengths of 30 mm or 60 mm. In addition, appropriately modified fluted tibial rods can be provided of lengths between 75 mm and 150 mm and diameters between 10 mm and 24 mm.

In one preferred embodiment, the comprehensive modular implant system is provided to the orthopaedic surgeon in a kit. The kit can include all of the components necessary to perform any of the replacement surgeries described above. The components can be assembled in the operating room, if necessary.

## Claims

1. A modular orthopaedic implant system comprising:
at least three components, at least one component configured for replacement of a portion of the bone of a limb of a patient, and at least another component configured for implantation within a remaining portion of the bone; and
a male-female engagement between adjacent ones of each of said at least three components.

2. A system as claimed in claim 1, wherein said male-female engagement is a Morse taper engagement.

3. A system as claimed in claim 1, wherein said at least one component includes a joint component configured to form part of a skeletal joint.

4. A system as claimed in claim 3, wherein said male-female engagement is a Morse taper engagement with said joint component including the female element of said Morse taper engagement.

5. A system as claimed in claim 1, wherein said at least another component includes a stem configured for implantation within the intra medullary canal of the remaining bone.

6. A system as claimed in claim 1, wherein said at least one component includes an elongated segmental component selected from a plurality of segmental components having different lengths.

7. A system as claimed in claim 6, wherein said male-female engagement is a Morse taper engagement and said segmental component includes a female element of said Morse taper at one end and a male element of said Morse taper at an opposite end.

8. A system as claimed in claim 6, wherein said at least one component includes a second segmental component selected from said plurality of segmental components having different lengths.

9. A system as claimed in claim 6, wherein said plurality of segmental components includes a first group of segmental components having lengths of 25 mm, 30 mm, 35 mm, 40 mm and 45mm.

10. A system as claimed in claim 9, wherein said plurality of segmental components includes a second group of segmental components having lengths of 65 mm, 85 mm, 105 mm and 125 mm, whereby a segmental component selected from said first group can be combined with a segmental component selected from either said first group or said second group to produce a combined length from 50 mm to 170 mm in 5 mm increments.

11. A system as claimed in claim 1, wherein said at least three components includes only pair of stems configured for implantation within proximal and distal portions of the bone, and a spacer disposed between said pair of stems.

12. A system as claimed in claim 1, wherein said male-female engagement includes a number of interlocking tabs and notches to prevent relative rotation between adjacent ones of said components.

13. A modular orthopaedic implant system for total replacement of the bone of a limb of a patient, comprising:
a first joint component configured to form part of a skeletal joint at the proximal end of the limb;
a second joint component configured to form part of a skeletal joint at the distal end of the limb;
at least two intermediate components spanning the length of the limb between said first and second joint components; and
a male-female engagement between adjacent one of said joint and intermediate components.

14. A system as claimed in claim 13, wherein said at least two intermediate components includes two elongated components selected from a plurality of elongated components having different lengths.

15. A system as claimed in claim 14, wherein said male-female engagement is a Morse taper engagement and said two elongated components both include a female element of said Morse taper engagement at one end and a male element of said Morse taper engagement at an opposite end.

16. A system as claimed in claim 14, wherein said at least two intermediate components includes a second elongated component selected from said plurality of elongated components having different lengths.

17. A system as claimed in claim 14, wherein said plurality of elongated components includes a first group of elongated components having lengths of 25 mm, 30 mm, 35 mm, 40 mm and 45mm.

18. A system as claimed in claim 17, wherein said plurality of elongated components includes a second group of elongated components having lengths of 65 mm, 85 mm, 105 mm and 125 mm, whereby an elongated component selected from said first group can be combined with an elongated component selected from either said first group or said second group to produce a combined length from 50mm to 170mm in 5mm increments.

19. A system as claimed in claim 14, wherein said at least two intermediate components further includes an elongated spacer disposed between one of said joint components and one of said two elongated components.

20. A system as claimed in claim 19, wherein:
said male-female engagement is a Morse taper engagement;
said two elongated components both include a female element of said Morse taper engagement at one end and a male element of said Morse taper engagement at an opposite end; and
said spacer component includes a male element of said Morse taper engagement at its ends.
